# EUROPEAN PATENT SPECIFICATION

(11) **EP 1 072 238 B1**
(45) Date of publication and mention of the grant of the patent: **26.11.2003**
(21) Application number: 00903705.2
(22) Date of filing: 11.02.2000
(51) Int. Cl.: A61F 2/48

(54) **EXTERNAL MAGNETIC ACTUATION VALVE FOR INTRAURETHRAL ARTIFICIAL URINARY SPHINCTER**
EXTERNES, MAGNETISCHES BETÄTIGUNGSVENTIL FÜR EINEN KÜNSTLICHEN INTRAURETHALEN SPHINKTER
VALVULE A ACTIONNEMENT MAGNETIQUE EXTERNE POUR UN SPHINCTER ARTIFICIEL INTRA-UTERIN

(30) Priority: 11.02.1999 ES 9900284
(43) Date of publication of application: 31.01.2001
(73) Proprietor: UNIVERSIDAD COMPLUTENSE DE MADRID, E-28040 Madrid (ES)
(72) Inventor: RIVERO RODRIGUEZ, Guillermo, E-28230 Madrid (ES); MULTIGNER DOMINGUEZ, Marta, E-28230 Madrid (ES); GARCIA PAEZ, José Maria, E-28035 Madrid (ES); CARBALLIDO RODRIGUEZ, Joaquin, E-28035 Madrid (ES); JORGE HERRERO, Eduardo, E-28035 Madrid (ES); TENDILLO CORTIJO, Francisco Javier, E-28035 Madrid (ES)
(74) Representative: Carpintero Lopez, Francisco
(86) International application number: PCT/ES00/00049
(87) International publication number: WO 00/047141

(56) References cited:
- EP-A1- 0 182 409
- EP-A1- 0 357 846
- WO-A1-95/17862
- WO-A1-95/29716
- WO-A1-99/18885
- WO-A1-99/51293
- ES-A6- 2 025 946
- FR-A1- 2 655 536
- US-A- 4 994 019
- US-A- 5 562 598

## Description

### OBJECT OF THE INVENTION

The present invention relates to a valve model for an artificial urinary sphincter to be placed intraurethrally in humans, which performs the functions of the external urinary sphincter, meant for treating urinary incontinence and/or retention. Opening of said valve is controlled from outside the human body by the application of a magnetic field created by a permanent magnet. The valve is closed automatically by a permanent magnet placed inside the valve.

The purpose of the present invention is the recovery of self-control of urination for those people who have lost it by means of an easily implanted prosthesis.

### BACKGROUND OF THE INVENTION

Hitherto, people suffering from urinary incontinence or retention deal with said problem with soakers, or probes provided with urine collection bags. The former is uncomfortable and causes serious social problems, while the latter often causes infections, often with a fatal outcome due to kidney failure. Work has been directed for some time towards designing prostheses which may replace the function of the urinary sphincter, although there is none yet in widespread use.

There exist some prosthesis which involve voluntary strangulation of the urethra (patent nos. US3744063, US3854469, US3903849, EP0314258). The one used most has been commercialized under the name AMS-800 of American Medical System. Implantation of this type of prosthesis requires invasive surgery. Additionally, the firm Uroquest has developed a system to fight urinary incontinence consisting of a probe with an outlet orifice at the end of the urethra, but which does not reach the exterior and which is opened by means of an external magnet.

An external magnetically actuated valve according to the preamble of claim 1, is known from the document WO-A- 95 29716.

The device object of the present invention allows control of urination by the patient and may be implanted without need of surgery by cytoscopy.

### DESCRIPTION OF THE INVENTION

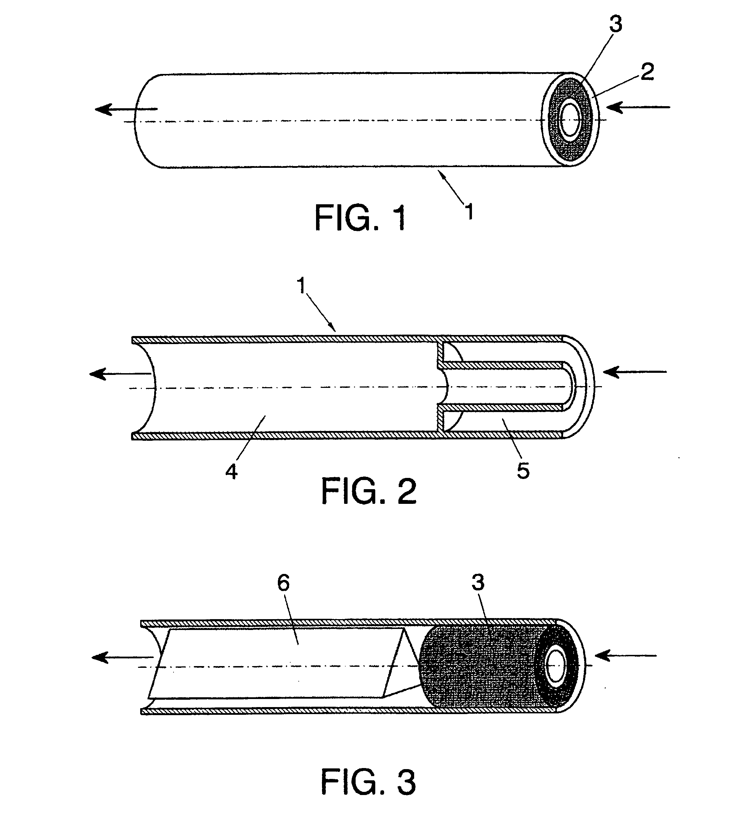

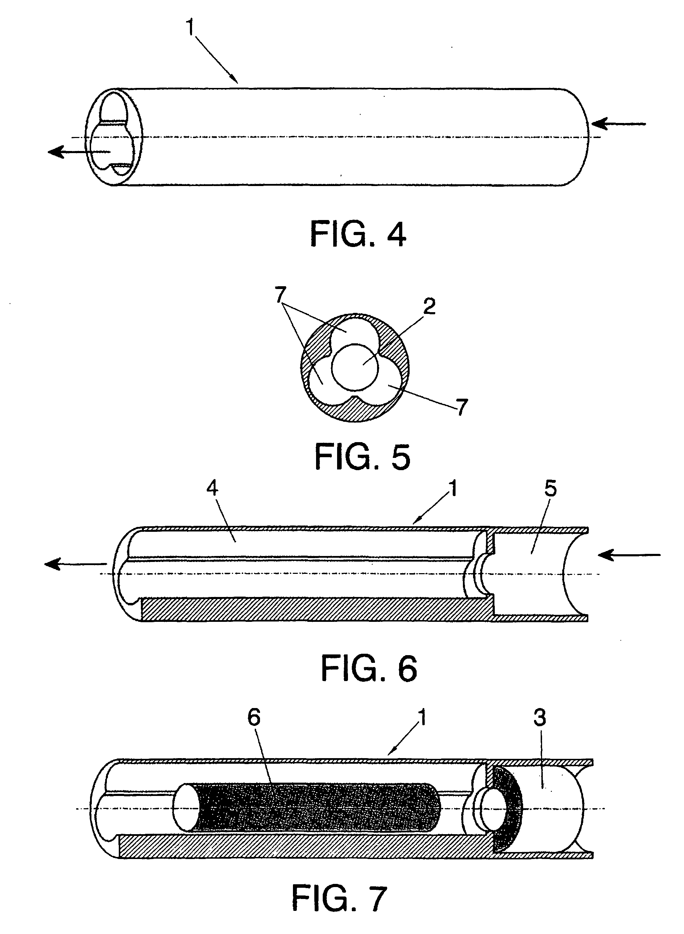

The external magnetically actuated valve for an artificial intraurethral sphincter is in essence a tightly sealed fluid valve provided with a securing system similar to that used for urethral probes, which may be implanted by cytoscopy. Two valve models are proposed, named "valve 1" and "valve 2", with different geometry but with the same working principle as described above:
The valves comprise ( figures 1 and 4):
   1. A hollow cylindrical body (1), made of a biocompatible material (e.g. Teflon) which forms the valve body, so that a fluid may pass through it.
   2. A piston (6) which may move within the cylindrical body. made of a soft magnetic material (e.g. Fe-Si 3%) coated in a biocompatible material (e.g. Teflon). One of its ends is in the form of a cone or hemisphere in order to improve the seal of the cylinder inlet orifice (2).
   3. A permanent magnet (3) (e.g.: NdFeB), coated in a biocompatible material (e.g. Teflon) attached to the inlet orifice of the valve cylindrical body.
The operation, as seen in figure 3, is as follows:
   Permanent magnet (3) attracts internal piston (6) so that when the cone-shaped piston end rests on inlet orifice (2) of the cylindrical valve body (1) the latter is closed.

To open the valve an external magnet (11) is used. When said magnet approaches the area where the valve is implanted from outside the human body, it exerts on piston (6) a greater attraction than that of internal toroidal magnet (3) in an opposite sense, due to the reorientation of the magnetic moment of piston (6), resulting in the valve opening and urine being released outwards. When outer magnet (6) is removed, the force exerted by toroidal magnet (3) on piston (6) again closes the valve inlet orifice shutting off urine flow outwards.

This valve is further provided with a safety system based on the weakening of the magnetic field with distance. After choosing a soft magnetic material for piston (6) and a hard one for the internal toroidal magnet (3), and depending on the distance which separates these two, there will always be a certain pressure exerted on the piston by urine which will result in the automatic opening of the valve. Thus it is ensured that if an external magnetic field cannot be applied for whichever reason, pressures above a certain limit will not be reached in the bladder, thus preventing damage to the urinary system from excessive pressure in the bladder.

### DESCRIPTION OF THE DRAWINGS

Figure 1 shows valve 1 and consists of a hollow cylindrical body (1), on one of whose bases (2) at the inlet orifice is placed a permanent magnet (3) so that a fluid may pass through it.
Figure 2. Shows a longitudinal section of cylindrical body (1) showing the space in which are placed piston (4) and permanent magnet (5).
Figure 3. Shows a longitudinal section of the cylindrical body containing a piston (6) and a permanent magnet (3).
Figure 4. Shows the hollow cylindrical body (1) of valve 2.
Figure 5. Shows a cross section of the hollow cylindrical body of figure 4 revealing the fluid inlet orifice (2) and the three lateral fluid outlet orifices (7).
Figure 6. Shows a longitudinal section of hollow cylindrical body (1) of valve 2 showing the space in which piston (4) and permanent magnet (5) are placed.
Figure 7. Shows a longitudinal section of valve 2, with cylindrical body (1), internal piston (6) and permanent magnet (3).

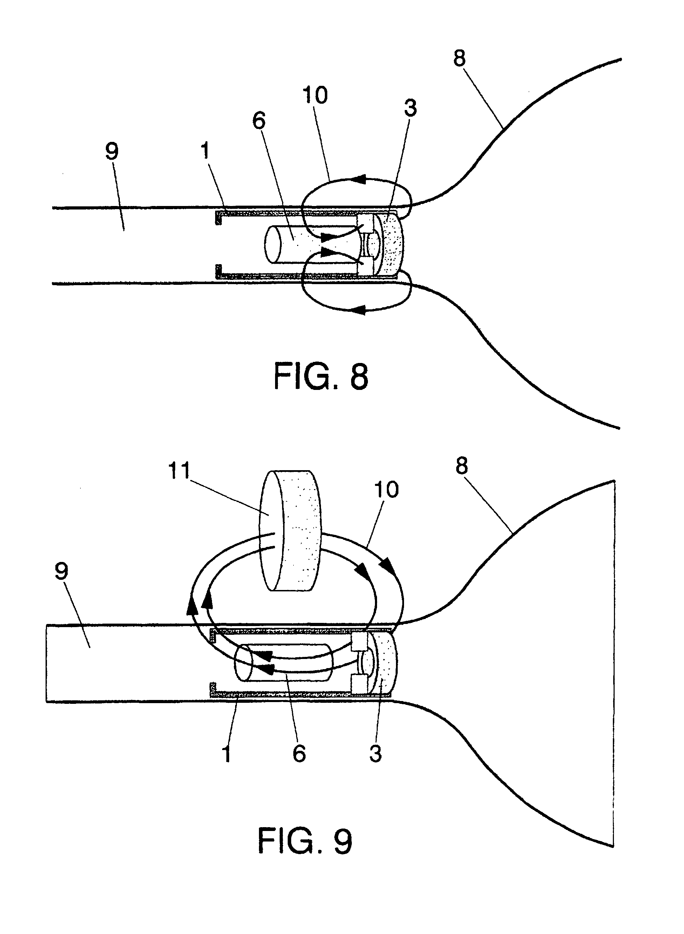

Figure 8. Shows an operation diagram for the closed valve, where (1) is the cylindrical valve body, (6) is the piston, (3) is the internal permanent magnet, (8) is the bladder, (9) is the urethra and (10) are the magnetic field lines of magnet (3).
Figure 9. Shows an operation diagram for the open valve, where (1) is the cylindrical valve body, (6) is the piston, (3) is the internal permanent magnet, (8) is the bladder, (9) is the urethra, (11) is the external permanent magnet and (10) are the magnetic field lines of magnet (11).

### PREFERRED EMBODIMENT OF THE INVENTION

The present invention relates to an external magnetically actuated valve for an artificial intraurethral urinary sphincter and is illustrated by the following examples:

Six different prototypes of the device have been constructed. The soft magntic materials used were sweet Fe, Fe-Si and Fe-P. The hard magnetic materiasl were magnets of NdFeB, SmCo and AlNiCo. These prototypes were tested *in vitro* in a hydrodynamical facility in which the tight seal, functioning of teh safety system, opening of the valve by applicaiton of an external magnetic field, evacuation flow and automatic closing were tested.

For *in vivo* tests two identical prototypes were constructed. A hollow cylindrical body which conforms the valve body was made in Teflon. Said body is provided with a cavity in the inlet orifice in which is placed the internal magnet under pressure. The permanent internal magnet is made of NdFeB and is coated in Teflon to avoid biologic incompatibilities. The pistons, which have a triangular base and a cone-shaped end are made of sweet Fe and coated in Teflon for the same reasons as above. The external permanent magnet is made of NdFeB.

The prototypes were inserted by urologist surgeons using surgical techniques in two pigs, one male and one female, in which a chronic urinary incontinence had been previously caused. Tight seal of the valve was tested to a pressure of 40 cm. H₂O. When the external magnet was placed near the valve, which was already in teh animal's urethra, the animal's urine was evacuated.

## Claims

1. External magnetically actuated valve for an artificial intraurethral urinary sphincter, comprising:
- a hollow cylindrical body (1), made of a biocompatible material.
- a piston (6), made from a magnetic material, with one end in the shape of a cone or hemisphere, which moves within the inside of the cylindrical body, caracterised in that it further comprises an internal permanent toroidal magnet (3) placed in the inlet orifice of the valve cylindrical body.

2. External magnetically actuated valve for an artificial intraurethral urinary sphincter, as claimed in above claim, **characterized in that** the piston may have a triangular or circular section.

3. External magnetically actuated valve for an artificial intraurethral urinary sphincter, as claimed in claim 1, **characterized in that** the valve is closed by the attraction exerted by the permanent magnet on the internal piston.

4. External magnetically actuated valve for an artificial intraurethral urinary sphincter, as claimed in above claims, **characterized in that** the valve is opened from the outside by approaching an external magnet, which exerts on the piston a greater force than the internal toroidal magnet and in the opposite sense, allowing urine to pass outwards.

5. External magnetically actuated valve for an artificial intraurethral urinary sphincter, as claimed in above claims, **characterized in that** it is provided with a safety system based on the decreasing force exerted between the internal magnet and the piston as the magnetic field decreases with increasing distance.

## Patentansprüche

1. Magnetisches externes Auslöseventil für einen künstlichen intraurethralen Urinsphinkter, der sich aus Folgendem zusammensetzt:
- einem hohlen zylindrischen Körper (1), der aus biokompatiblem Material hergestellt ist.
- einem Kolben (6), der aus , magnetischem Material hergestellt ist und dessen eines Ende kegelförmig oder halbrund ausgebildet ist und sich im Inneren des zylindrischen Körpers bewegt, **dadurch gekennzeichnet, dass** er ausserdem über einen permanenten ringförmigen Magneten (3), der an der Eintrittsöffnung des zylindrischen Körpers des Ventils befestigt ist, verfügt.

2. Magnetisches externes Auslöseventil für einen künstlichen intraurethralen Urinsphinkter, gemäss des vorangehenden Anspruchs, **dadurch gekennzeichnet, dass** der Kolben einen dreieckigen oder runden Querschnitt aufweist.

3. Magnetisches externes Auslöseventil für einen künstlichen intraurethralen Urinsphinkter, gemäss Anspruch 1, **dadurch gekennzeichnet, dass** das Schliessen des Ventils durch die Anziehungskraft stattfindet, die der permanente Magnet auf den inneren Kolben ausübt.

4. Magnetisches externes Auslöseventil für einen künstlichen intraurethralen Urinsphinkter, gemäss den vorangehenden Ansprüchen, **dadurch gekennzeichnet, dass** die Öffnung des Ventils von Aussen durch die Annäherung eines externen Magnets stattfindet, der auf den Kolben eine höhere Kraft ausübt, als die des inneren ringförmigen Magnets und in die umgekehrte Richtung, so dass der Urin nach Aussen gelangen kann.

5. Magnetisches externes Auslöseventil für einen künstlichen intraurethralen Urinsphinkter, gemäss den vorangehenden Ansprüchen, **dadurch gekennzeichnet, dass** es über ein Sicherheitssystem verfügt, dass auf der abnehmenden Kraft zwischen dem internen Magneten und dem Kolben beruht, da das Magnetfeld mit zunehmender Entfernung abnimmt.

## Revendications

1. Valve de commande magnétique externe pour sphincter urinaire artificiel intra-urétral, comprenant:
- un corps cylindrique creux (1), fabriqué en matière biocompatible.
- un piston (6), fabriqué en matière magnétique, en ayant une de ses extrémités terminée sous forme de cône ou de demi-sphère, qui est déplacé à l'intérieur du corps cylindrique, **caractérisé en ce qu'**il comprend en outre, un aimant toroïdal permanent (3) interne fixé dans l'orifice d'entrée du corps cylindrique de la valve.

2. Valve de commande magnétique externe pour un sphincter urinaire artificiel intra-urétral, selon la revendication précédente **caractérisée en ce que** le piston peut être de section triangulaire ou circulaire.

3. Valve de commande magnétique externe pour un sphincter urinaire artificiel intra-urétral, selon la revendication 1, **caractérisée en ce que** la fermeture de la valve est réalisée au moyen de la force attractive exercée par l'aimant permanent sur le piston interne.

4. Valve de commande magnétique externe pour un sphincter urinaire artificiel intra-urétral, selon les revendications précédentes, **caractérisée en ce que** l'ouverture de la valve depuis l'extérieur est réalisée au moyen de l'approchement d'un aimant externe, en exerçant sur le piston une force plus intense que celle de l'aimant toroïdal interne et dans le sens inverse, en permettant le passage de l'urine vers l'extérieur.

5. Valve de commande magnétique externe pour un sphincter urinaire artificiel intra-urétral, selon les revendications précédentes, **caractérisée en ce qu'**elle dispose d'un système de sécurité basé sur la diminution de la force entre l'aimant interne et le piston lors de la réduction du champ magnétique due à l'augmentation de la distance.
